# EUROPÄISCHE PATENTSCHRIFT

(11) **EP 0 716 648 B1**
(45) Veröffentlichungstag und Bekanntmachung des Hinweises auf die Patenterteilung: **28.10.1998**
(21) Anmeldenummer: 94927509.3
(22) Anmeldetag: 29.08.1994
(51) Int. Cl.: C07C 405/00, A61K 31/557

(54) **NEUE 9-CHLOR-PROSTAGLANDIN-DERIVATE**
NEW 9-CHLORO-PROSTAGLANDIN DERIVATIVES
NOUVEAUX DERIVES DE 9-CHLOROPROSTAGLANDINE

(30) Priorität: 31.08.1993 DE 4330177
(43) Veröffentlichungstag der Anmeldung: 19.06.1996
(73) Patentinhaber: SCHERING AKTIENGESELLSCHAFT, 13353 Berlin (DE)
(72) Erfinder: BUCHMANN, Bernd, D-16540 Hohen Neuendorf (DE); SKUBALLA, Werner, D-13465 Berlin (DE)
(86) Internationale Anmeldenummer: EP9402855
(87) Internationale Veröffentlichungsnummer: WO9506634

(56) Entgegenhaltungen:
- EP-A- 0 030 377
- WO-A-94/08585
- DE-A- 4 229 048

## Beschreibung

Gegenstand der Erfindung sind 9-Chlor-prostaglandinanaloga, Verfahren zu ihrer Herstellung sowie ihre Verwendung als Arzneimittel.

Aus dem umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch auf Schwierigkeiten. Die meisten natürlichen Prostaglandine besitzen eine für therapeutische Zwecke zu kurze Wirkdauer, da sie zu rasch durch verschiedene enzymatische Prozesse abgebaut werden. Alle Strukturveränderungen haben daher das Ziel, sowohl die Wirkdauer als auch die Selektivität der Wirkung zu steigern.

In der europäischen Patentanmeldung EP 030 377 A1 werden bereits 9-Chlorprostaglandinderivate, Verfahren zu deren Herstellung und ihre Verwendung als Arzneimittel offenbart. Die genannten Verbindungen können eine Menstruation induzieren oder eine Schwangerschaft unterbrechen. Auch weitere Anwendungen, wie z.B. die Synchronisation des Sexualcyclus weiblicher Säugetiere, werden genannt.

Es wurde nun gefunden, daß neue 9-Chlor-prostaglandinanaloga eine hohe Wirkspezifität, bessere Wirksamkeit, längere Wirkdauer und vor allen Dingen höhere Stabilität als die natürlichen Prostaglandine besitzen.

Die Erfindung betrifft 9-Chlor-prostaglandin-derivate der Formel I 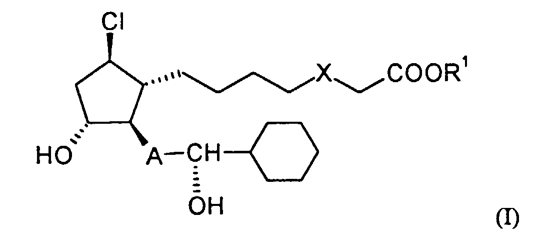 worin
- X: Sauerstoff oder CH₂,
- R¹: Wasserstoff oder geradkettiges oder verzweigtes Alkyl mit 1-6 C-Atomen und
- A: Trans-CH=CH- oder -CH₂-CH₂₋
bedeuten, sowie deren Salze mit physiologisch verträglichen Basen, wenn R¹ Wasserstoff darstellt und deren Clathrate mit α-, β- oder γ-Cyclodextrin.

Als physiologisch verträgliche Basen kommen z. B. Alkalilaugen wie KOH, NaOH oder Erdalkalihydroxide wie Ca(OH)₂ in Betracht.

Die Erfindung betrifft ferner ein Verfahren zur Herstellung der 9-Chlorprostaglandinanaloga der Formel I, dadurch gekennzeichnet, daß man
a) eine Verbindung der Formel II 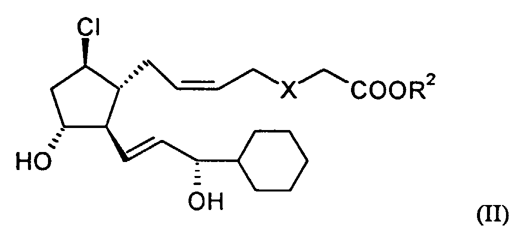 worin
   R² eine geradkettige oder verzweigte Alkylgruppe mit 1-6 C-Atomen und
   X die oben angegebene Bedeutung aufweisen, nach Schutz der OH-Gruppen mit Dimethyl-tert.-butylsilylchlorid in Gegenwart von Pd/C hydriert und anschließend die Schutzgruppen mit Tetrabutylammoniumfluorid-trihydrat entfernt oder
b) eine Verbindung der Formel II in Gegenwart von Pd/C hydriert
und gegebenenfalls die nach Variante a) oder b) erhaltenen Verbindungen mit Alkalilauge verseift und die so erhaltenen Verbindungen der allgemeinen Formel I mit R¹ = H falls gewünscht mit Diazoalkanen der Formel III

R³N₂ (III),

worin R³ eine Alkylgruppe mit 1-6 C-Atomen bedeutet,
umsetzt.

Als Alkylrest R¹, R² und R³ kommen geradkettige oder verzweigte C₁-C₆-Alkyl-Reste wie Methyl, Ethyl, Propyl, Isopropyl, Butyl, Isobutyl, tert.-Butyl, Pentyl, Isopentyl, Neopentyl, Hexyl, Isohexyl u.s.w. in Betracht.

Die Erfindung betrifft auch Arzneimittel auf Basis der Verbindungen der Formel I, sowie deren Cyclodextrinclathrate, mit den üblichen Hilfs- und Trägerstoffen.

Cyclodextrinclathrate können analog einer Vorschrift in WO 87/05294 erhalten werden.

Die erfindungsgemäßen 9-Chlor-prostaglandinanaloga sind stabile PGD₂-Derivate und damit wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Sie sind als medizinisch wertvolle Wirkstoffe zur Anwendung für z.B. Blutdrucksenkung, die Förderung der Hautdurchblutung, Luteolyse, Hemmung der Magensäuresekretion, Plättchenaggregationshemmung, Ulkusheilung oder Zytoprotektion geeignet.

Die erfindungsgemäßen Verbindungen können auch in Kombination, z.B. mit β-Biockern. Diuretika, Phosphodiesterasehemmern, Calciumantagonisten, Thromboxanantagonisten, Thromboxansynthetase- und Cyclooxygenasehemmern, gerinnungshemmenden Substanzen, wie auch Fibrinolytika, Leukotrienantagonisten, Leukotriensynthetasehemmern und Antigestagenen, verwendet werden.

Besonders geeignet sind die erfindungsgemäßen Verbindungen zur lokalen Anwendung wie z.B. zur Förderung der Hautdurchblutung und zur Senkung erhöhten Augeninnendruckes (Glaukom), sowie zur Förderung der Nierendurchblutung und zur Verwendung als Diuretikum.

Bei Kaninchen bewirkt die lokale Applikation der Verbindungen eine Senkung des Augeninnendruckes.
Bei Affen mit experimentellem Glaukom bewirkt die lokale Applikation der Verbindungen eine Normalisierung des pathologisch erhöhten Augeninnendruckes.
Die Einzeldosis der Verbindungen für die Anwendung zur Behandlung erhöhten Augeninnendruckes ist 1 ng - 100 µg / Auge, einmal oder mehrmals täglich, wenn sie am menschlichen Patienten lokal verabreicht werden.
Für die lokale Applikation, wie z. B. für die Anwendung zur Behandlung erhöhten Augeninnendruckes sind beispielsweise Lösungen, Lotionen oder Salben geeignet.

Die Dosis der Verbindungen bei lokaler Anwendung zur Förderung der Hautdurchblutung ist 5-500 ng/cm², wenn sie am menschlichen Patienten verabreicht werden.

Für die lokale Applikation für die Anwendung zur Förderung der Hautdurchblutung sind beispielsweise Lösungen, Lotionen, Salben, Cremes oder Pflaster geeignet.

Die erfindungsgemäßen Wirkstoffe sollen in Verbindung mit den in der Galenik bekannten und üblichen Hilfsstoffen, z.B. zur Herstellung von Präparaten zur Förderung der Hautdurchblutung, zur Behandlung des erhöhten Augeninnendruckes (Glaukom), zur Förderung der Nierendurchblutung oder zur Verwendung als Diuretikum dienen.

Die folgenden Beispiele sollen die Erfindung näher erläutern, ohne daß damit eine Begrenzung vorgenommen werden soll.

### Beispiel 1

### (13E)-(9R,11R,15R)-9-Chlor-3-oxa-15-cyclohexyl-11,15-dihydroxy-16,17,18,19,20-pentanor-13-prostensäure-tert.-butylester

Zu einer Lösung von 125 mg (13E)-(9R,11R,15R)-9-Chlor-3-oxa-15-cyclohexyl-11,15-bis-(tert.-butyldimethylsilyloxy)-16,17,18,19,20-pentanor-13-prostensäure-tert.-butylester aus Beispiel 1b) in 3 ml Tetrahydrofuran gibt man bei 24°C 315 mg Tetrabutylammoniumfluorid-trihydrat und rührt bei dieser Temperatur für 30 Stunden. Anschließend wird mit Diethylether verdünnt, einmal mit Wasser gewaschen und über Natriumsulfat getrocknet. Nach Filtration wird im Vakuum eingeengt. Den so erhaltenen Rückstand reinigt man durch Chromatographie an Kieselgel. Mit Hexan / 0-100% Diethylether erhält man 71 mg der Titelverbindung als farbloses Öl.
IR (Film): 3404, 2927, 2854, 1748, 1450, 1368,1229, 1137, 845 cm⁻¹.

Das Ausgangmaterial für die obige Titelverbindung wird wie folgt hergestellt:

1a) (5Z,13E)-(9R,11R,15R)-9-Chlor-3-oxa-15-cyclohexyl-11,15-bis-(tert.-butyldimethylsilyloxy)-16,17,18,19,20-pentanor-5,13-prostadiensäure-tert.-butylester Zu einer Lösung von 100 mg (5Z,13E)-(9R,11R,15R)-9-Chlor-3-oxa-15-cyclohexyl-11,15-dihydroxy-16,17,18,19,20-pentanor-5,13-prostadiensäure-tert.-butylester in 3 ml Dimethylformamid gibt man bei 0°C unter Argon nacheinander 136 mg Imidazol und 150 mg tert.-Butyldimethylsilylchlorid. Nach 20 Stunden Rühren bei 24°C wird mit Diethylether verdünnt, mit Wasser gewaschen, über Natriumsulfat getrocknet und nach Filtration im Vakuum eingeengt. Den so erhaltenen Rückstand reinigt man durch Chromatographie an Kieselgel. Mit Hexan / 5-10% Diethylether erhält man 135 mg der Titelverbindung als farbloses Öl.
IR (Film): 2928, 2855, 1750, 1472, 1368, 1255, 1127, 836, 775 cm⁻¹.

1b) (13E)-(9R,11R,15R)-9-Chlor-3-oxa-15-cyclohexyl-11,15-bis-(tert.-butyldimethylsilyloxy)-16,17,18,19,20-pentanor-13-prostensäure-tert.-butylester 125 mg des unter 1a) hergestellten Bis-Silylethers werden in 20 ml Essigester gelöst und 2 Stunden in einer Wasserstoffatmosphäre mit 12.5 mg Palladium (10%) auf Kohle bei 24°C gerührt. Anschließend wird vom Katalysator abfiltriert und im Vakuum eingeengt. Die so erhaltenen 125 mg der Titelverbindung werden ohne weitere Reinigung in die nächste Stufe eingesetzt.
IR (Film): 2928, 2855, 1752, 1472,1368, 1255, 1137, 1006, 972, 899, 836, 775 cm⁻¹.

### Beispiel 2

### (9R,11R,15R)-9-Chlor-3-oxa-15-cyclohexyl-11,15-dihydroxy-16,17,18,19,20-pentanorprostansäure-tert.-butylester

Eine Lösung von 100 mg (5Z,13E)-(9R,11R,15R)-9-Chlor-3-oxa-15-cyclohexyl-11,15-dihydroxy-16,17,18,19,20-pentanor-5,13-prostadiensäure-tert.-butylester in 15 ml Essigester wird für 2.5 Stunden mit 10 mg Palladium (10%) auf Kohle in einer Wasserstoffatmosphäre bei 24°C gerührt. Nach Filtration wird im Vakuum eingeengt. Den so erhaltenen Rückstand reinigt man durch Chromatographie an Kieselgel. Mit Toluol / 5% Isopropanol erhält man 92 mg der Titelverbindung als farbloses Öl.
IR (Film): 3397, 2925, 2853, 1748, 1496, 1450, 1368, 1241, 1136, 970, 846, 730 cm⁻¹.

### Beispiel 3

### (13E)-(9R,11R,15R)-9-Chlor-3-oxa-15-cyclohexyl-11,15-dihydroxy-16,17,18,19,20-pentanor-13-prostensäure

Zu einer Lösung aus 60 mg des in Beispiel 1) hergestellten Esters in 1 ml Methanol gibt man 1 ml 0.5 N Natronlauge und rührt 4 Tage bei 24°C unter Argon. Man säuert anschließend auf einen ph-Wert von 4 mit 1 N Schwefelsäure an und extrahiert mit Essigester. Die organische Phase wird mit gesättigter Natriumchlorid-Lösung gewaschen, über Natriumsulfat getrocknet und nach Filtration im Vakuum eingeengt. Man erhält auf diese Weise 36 mg der Titelverbindung als Öl.
IR (CHCl₃): 3402, 2928, 2855, 1732, 1450, 1135, 973 cm⁻¹.

### Beispiel 4

### (9R,11R,15R)-9-Chlor-3-oxa-15-cyclohexyl-11,15-dihydroxy-16,17,18,19,20-pentanorprostansäure

In Analogie zu Beispiel 3 erhält man aus 75 mg des in Beispiel 2) hergestellten Esters 56 mg der Titelverbindung als Öl.
IR (CHCl₃): 3418, 2930, 2855, 1732, 1450, 1135 cm⁻¹.

### Beispiel 5

### (13E)-(9R,11R,15R)-9-Chlor-3-oxa-15-cyclohexyl-11,15-dihydroxy-16,17,18,19,20-pentanor-13-prostensäuremethylester

Zu einer Lösung aus 18 mg der in Beispiel 3) hergestellten Säure in 2 ml Methylenchlorid gibt man bei 0°C unter Argon eine ethensche Diazomethanlösung bis eine bleibende Gelbfärbung erkennbar ist. Anschließend wird im Vakuum eingeengt. Man erhält auf diese Weise 18 mg der Titelverbindung als Öl.
IR (CHCl₃): 3414, 2928, 2855, 1734, 1448, 1374, 1110, 1045, 1002, 892 cm⁻¹.

### Beispiel 6

### (9R,11R,15R)-9-Chlor-3-oxa-15-cyclohexyl-11,15-dihydroxy-16,17,18,19,20-pentanorprostansäureethylester

In Analogie zu Beispiel 5) erhält man aus 15 mg der in Beispiel 4) hergestellten Säure mit etherischer Diazoethanlösung 16 mg der Titelverbindung als Öl.
IR (CHCl₃): 3413, 2930, 2855, 1740, 1450, 1378, 1262, 1188, 1136, 996, cm⁻¹.

## Patentansprüche

1. 9-Chlor-prostaglandin-derivate der Formel I 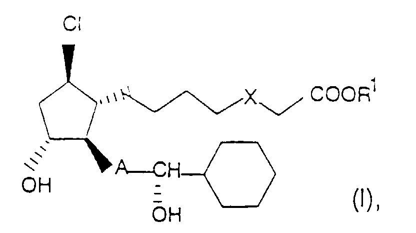 worin
X Sauerstoff oder CH₂,
R¹ Wasserstoff oder geradkettiges oder verzweigtes Alkyl mit 1-6 C-Atomen und
A trans-CH=CH- oder -CH₂-CH₂-
bedeuten, sowie deren Salze mit physiologisch verträglichen Basen, wenn R¹ Wasserstoff darstellt und deren Clathrate mit α-, β- oder γ-Cyclodextrin.

2. (13E)-(9R,11R,15R)-9-Chlor-3-oxa-15-cyclohexyl-11,15-dihydroxy-16,17,18,19,20-pentanor-13-prostensäure-tert.-butylester

3. (9R,11R,15R)-9-Chlor-3-oxa-15-cyclohexyl-11,15-dihydroxy-16,17,18,19,20-pentanorprostansäure-tert.-butylester

4. (13E)-(9R,11R,15R)-9-Chlor-3-oxa-15-cyclohexyl-11,15-dihydroxy-16,17,18,19,20-pentanor-13-prostensäure

5. (9R,11R,15R)-9-Chlor-3-oxa-15-cyclohexyl-11,15-dihydroxy-16,17,18,19,20-pentanorprostansäure

6. (13E)-(9R,11R,15R)-9-Chlor-3-oxa-15-cyclohexyl-11,15-dihydroxy-16,17,18,19,20-pentanor-13-prostensäuremethylester

7. (9R,11R,15R)-9-Chlor-3-oxa-15-cyclohexyl-11,15-dihydroxy-16,17,18,19,20-pentanorprostansäureethylester

8. Verfahren zur Herstellung der 9-Chlor-prostaglandinanaloga der Formel I, dadurch gekennzeichnet, daß man
a) eine Verbindung der Formel II 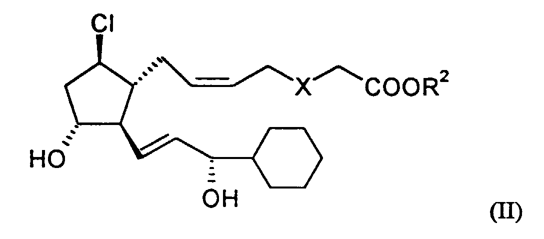 worin
R² eine geradkettige oder verzweigte Alkylgruppe mit 1-6 C-Atomen und
X die oben angegebene Bedeutung aufweisen, nach Schutz der OH-Gruppen mit Dimethyl-tert.-butylsilylchlorid in Gegenwart von Pd/C hydriert und anschließend die Schutzgruppen mit Tetrabutylammoniumfluorid-trihydrat entfernt oder
b) eine Verbindung der Formel II in Gegenwart von Pd/C hydriert
und gegebenenfalls die nach Variante a) oder b) erhaltenen Verbindungen mit Alkalilauge verseift und die so erhaltenen Verbindungen der allgemeinen Formel I mit R¹ = H falls gewünscht mit Diazoalkanen der Formel III
R³N₂ (III),
worin R³ eine Alkylgruppe mit 1-6 C-Atomen bedeutet,
umsetzt.

9. Arzneimittel auf Basis der Verbindungen der Formel I oder deren Cyclodextrinclathrate mit den üblichen Hilfs- und Trägerstoffen.

## Claims

1. 9-Chloro-prostaglandin derivatives of formula I 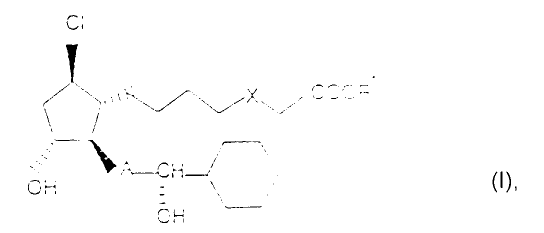 wherein
X represents oxygen or CH₂,
R¹ represents hydrogen or straight-chained or branched alkyl having from 1 to 6 carbon atoms and
A represents trans-CH=CH- or -CH₂-CH₂-,
and the salts thereof with physiologically tolerable bases when R¹ represents hydrogen and the clathrates thereof with α-, β- or γ-cyclodextrin.

2. (13E)-(9R,11R,15R)-9-Chloro-3-oxa-15-cyclohexyl-11,15-dihydroxy-16,17,18,19,20-pentanor-13-prostenoic acid tert-butyl ester.

3. (9R,11R,15R)-9-Chloro-3-oxa-15-cyclohexyl-11,15-dihydroxy-16,17,18,19,20-pentanor-prostanoic acid tert-butyl ester.

4. (13E)-(9R,11R,15R)-9-Chloro-3-oxa-15-cyclohexyl-11,15-dihydroxy-16,17,18,19,20-pentanor-13-prostenoic acid.

5. (9R, 11R,15R)-9-Chloro-3-oxa-15-cyclohexyl-11,15-dihydroxy-16,17,18,19,20-pentanor-prostanoic acid.

6. (13E)-(9R,11R,15R)-9-Chloro-3-oxa-15-cyclohexyl-11,15-dihydroxy-16,17,18,19,20-pentanor-13-prostenoic acid methyl ester.

7. (9R, 11R,15R)-9-Chloro-3-oxa-15-cyclohexyl-11,15-dihydroxy-16,17,18,19,20-pentanor-prostanoic acid ethyl ester.

8. Process for the preparation of 9-chloro-prostaglandin analogues of formula I, characterised in that
a) a compound of formula II 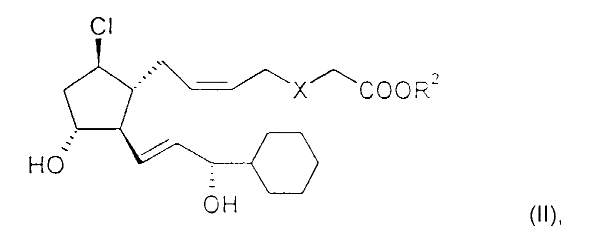 wherein
R² represents a straight-chained or branched alkyl group having from 1 to 6 carbon atoms and
X has the meaning mentioned above,
is, after protection of the OH groups using dimethyl-tert-butylsilyl chloride, hydrogenated in the presence of Pd/C and the protecting groups are then removed using tetrabutylammonium fluoride trihydrate or
b) a compound of formula II is hydrogenated in the presence of Pd/C,
and, optionally, the compounds obtained according to variant a) or b) are hydrolysed using alkali solution and the resulting compounds of the general formula I wherein
R¹ = H are reacted, if desired, with diazoalkanes of formula III
R³N₂ (III)
wherein R³ represents an alkyl group having from 1 to 6 carbon atoms.

9. Medicaments based on the compounds of formula I or the cyclodextrin clathrates thereof, together with the customary excipients and carriers.

## Revendications

1. Dérivés de 9-chloro-prostaglandines de formule I 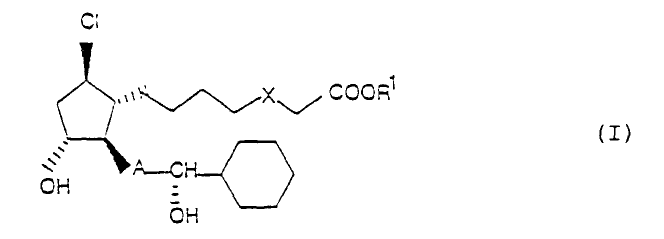 dans laquelle
X représente un atome d'oxygène ou CH₂,
R¹ représente un atome d'hydrogène ou un groupe alkyle linéaire ou ramifié avec 1 à 6 atomes de carbone, et
A représente un groupe trans-CH=CH- ou -CH₂-CH₂-,
ainsi que leurs sels avec des bases physiologiquement tolérées, lorsque R¹ représente un atome d'hydrogène et leurs clathrates avec l'α-, la β- ou la γ-cyclodextrine.

2. (13E)-(9R,11R,15R)-9-chloro-3-oxa-15-cyclohexyl-11,15-dihydroxy-16,17,18,19,20-pentanor-13-prosténoate de tert-butyle

3. (9R,11R,15R)-9-chloro-3-oxa-15-cyclohexyl-11,15-dihydroxy-16,17,18,19,20-pentanor-prostanoate de tert-butyle

4. Acide (13E)-(9R,11R,15R)-9-chloro-3-oxa-l5-cyclohexyl-11,15-dihydroxy-16,17,18,19,20-pentanor-13-prosténoïque

5. Acide (9R,11R,15R)-9-chloro-3-oxa-15-cyclohexyl-11,15-dihydroxy-16,17,18,19,20-pentanor-13-prostanoïque

6. (13E)-(9R,11R,15R)-9-chloro-3-oxa-15-cyclohexyl-11,15-dihydroxy-16,17,18,19,20-pentanor-13-prosténoate de méthyle

7. (9R,11R,15R)-9-chloro-3-oxa-15-cyclohexyl-11,15-dihydroxy-16,17,18,19,20-pentanor-prostanoate d'éthyle

8. Procédé pour la préparation des dérivés de 9-chloro-prostaglandines de formule I, caractérisé en ce que
a) on hydrogène un composé de formule II 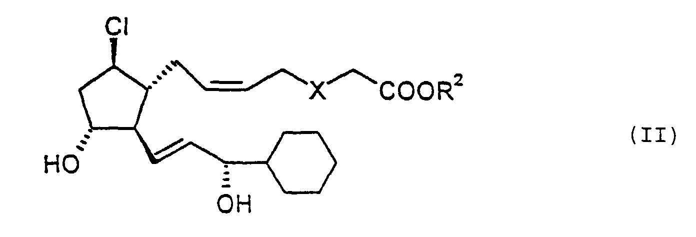 dans laquelle
R² représente un groupe alkyle linéaire ou ramifié avec 1 à 6 atomes de carbone et
X a la signification donnée ci-dessus, après avoir protégé les groupes OH avec du chlorure de diméthyl-tert-butylsilyle en présence d'un catalyseur Pd/C et ensuite on élimine les groupes protecteurs avec du fluorure de tétrabutylammonium trihydraté ou
b) on hydrogène un composé de formule II en présence d'un catalyseur Pd/C,
et éventuellement on saponifie avec une lessive alcaline les composés obtenus selon la variante a) ou b) et on fait réagir les composés de formule I ainsi obtenus avec R¹ = H si on le souhaite avec des diazoalcanes de formule III
R³N₂ (III)
dans laquelle R³ représente un groupe alkyle avec 1 à 6 atomes de carbone.

9. Médicaments à base des composés de formule I ou leurs clathrates de cyclodextrine avec des adjuvants et véhicules usuels.
